# EUROPEAN PATENT APPLICATION

(11) **EP 2 140 872 A2**
(43) Date of publication of application: **06.01.2010**
(21) Application number: 09164408.8
(22) Date of filing: 02.07.2009
(51) Int. Cl.: A61K 33/06, A61K 33/14, A61K 35/08, A61P 17/00

(54) **Composition for the treatment of skin conditions comprising sea water with additional magnesium**

(30) Priority: 02.07.2008 US 166605; 08.12.2008 US 330281
(71) Applicant: Wileford, Kenneth, Little Rive, SC 29566 (US)
(72) Inventor: Wileford, Kenneth, Little Rive, SC 29566 (US)
(74) Representative: Johnson, Yvonne Catherine

(57) **Abstract**

The present invention relates to artificial seawater and its use to treat a variety of skin conditions, when that sea water has more magnesium in it than in naturally occurring seawater. The treatment of acne, would healing and the like can be improved with the product of the present invention and with treatments thereof.

## Description

### COPYRIGHT NOTICE

A portion of the disclosure of this patent contains material that is subject to copyright protection. The copyright owner has no objection to the reproduction by anyone of the patent document or the patent disclosure as it appears in the Patent and Trademark Office patent files or records, but otherwise reserves all copyright rights whatsoever.

### BACKGROUND OF THE INVENTION

### Field of the Invention

The present invention relates to skin care. In particular the present invention relates to a composition that is useful in preventing or treating skin conditions, e.g. acne or other facial related infections or conditions, in the irrigation of wounds, in the prevention or treatment of hair loss, as a sunscreen, or as a substitute for saline in the treatment of a mammal.

### Description of Related Art

Many conditions of the skin are related to alterations in physiology and biochemistry. Trace amounts of minerals, elements, ions and metals are used in healthy skin metabolism for membrane function, immune modulation and enzyme co factors. Natural sea water contains a variety of inorganic salts in a combined concentration near 35 g/ Kg. Application of natural sea water to the skin is acknowledged to be helpful for skin conditions such as acne vulgaris. There does not appear to be information as to why sea water works the way it does, if any particular ingredient is the cause and if anything other than natural sea water has the effect. Artificial sea water can be manufactured to approximate the concentrations of many of the components in sea water although it does not contain all the ingredients of natural sea water.

Acne vulgaris is a multifactorial inflammatory disease affecting the pilosebaceous follicles. The pilosebaceous is composed of epidermal cells lining the hair follicle and the sebaceous gland. Each hair follicle is associated with one or more sebaceous glands. The infundibulum communicates directly with the epidermis and extends to the opening of the sebaceous gland. In a normal follicle, sebum is secreted from the sebaceous glands and carries desquamated keratinocytes from the follicular epithelium up the follicular canal toward the infundibulum. Keratinocytes are shed as single cells and are moved to the follicle lumen and removed. In acne vulgaris, however, keratinocytes hyperproliferate are shed as a group of cells rather than individual cells which obstruct the neck of the follicle. Acne vulgaris develops when the infundibulum becomes occluded, trapping sebum, shed cells and bacterial products, and leads to inflammation.

The pathogenesis of acne is only partially understood and is multifactorial. When abnormally desquamated keratinocytes accumulate in the sebaceous follicle, comedogenesis occurs and a microcomedo is formed. A microcomedo is a microscopic lesion that is not clinically evident but is the precursor of all acne lesions. Sebum flow is blocked and the follicle becomes plugged with lipids, bacteria, and cell fragments. The microcomedo enlarges and eventually becomes clinically visible. Noninflammatory lesions are an open or closed comedo. Inflammatory acne lesions can be a macule, papule, pustule or nodule.

Several factors have been well established in the pathogenesis of acne. These include sebum production, hormones, bacterial proliferation and inflammation. Sebum is a complex mixture of relatively non polar lipids that is secreted onto the surface of the skin by mature sebocytes. Excess sebum production is involved in the development of acne and is regulated by a number of factors, including local androgens. Both clinical observation and experimental evidence confirm the importance of hormones in the pathophysiology of acne. There is also the proliferation of the bacteria, Propionibacterium acnes, a Gram-positive anaerobe which induces inflammation by releasing lipases, proteases, hyaluronidases, and chemotactic factors. Immune cells are activated to induce an inflammatory response.

The proliferation of Propionibacterium acnes within the follicle is responsible for the release of various chemotactic and proinflammatoy mediators. There is a release of chemotactic substances that attract neutrophils, monocytes, and lymphocytes to the epithelial walls of sebaceous follicles, stimulate the production of proinflammatoy cytokines, activation of the complement system, and induces cell mediated immunity. The result of this intensified inflammatory cascade is the disruption of the follicular epithelium, leading to extravasation of lipids, keratinocytes, bacterial antigens, and inflammatory mediators into the surrounding dermis. Follicular rupture and secondary inflammatory responses are responsible for the progression of microcomedones to mature comedones or inflammatory acne lesions.

Many agents are used by physicians for the treatment of acne to focus on pathogenic targets. These agents have been used alone or in combination. Agents used for the hormonal component include estrogens, antiandrogens, and spironolactone. Topical and oral retinoids as well as alpha and beta hydroxyl acids have been used for hyperkeratinization. Sebum production has been treated with topical and oral retinoids, antiandrogens, laser therapy, and photodynamic therapy. Inflammation has been treated with topical and oral antibiotics as well as benzoyl peroxide. Antibacterial agents include topical and oral antibiotics, azelaic acid, benzoyl peroxide, and light therapy.

It is generally acknowledged that minerals support healthy skin metabolism. About 4-5% of the human body is made up of minerals. Many of these act as essential co-factors for enzymatic activity and in normal cellular membrane physiology. Of these minerals, magnesium has been the most widely studied. The precise mechanism and specific minerals involved in maintenance of homeostasis of skin has not been elucidated.

Topical application of natural sea water has also been used for the treatment of acne; however, the clinical effectiveness has not been studied in the medical literature. The mechanism of the effect is therefore not completely understood. Sea water is a complex mixture of dissolved minerals, metals and ions in a unique composition which varies from location to location. Natural sea water contains about 35 g/Kg of various inorganic salts.

Magnesium is the third most abundant mineral in sea water. Typically magnesium in seawater is present at a level of about 53 mM and frequently is reported as concentrations of 1000 ppm. It is known that higher concentrations of magnesium have a detrimental effect on sea life and, as such, high concentrations where there is aquatic life is not encountered. Studies have shown that elevated concentrations of magnesium have potentially negative biological effects.

Artificial sea water has been developed for use in aquariums. Professional marine biologists and reef aquarists are aware that artificial sea water is an imperfect substitute for the perfect medium for marine animal growth which is pure oceanic water. Attempts to reproduce the chemical composition of natural sea water have provided products that are commercially available. In general, these products make extensive attempts to reproduce the concentration of elements and minerals as they occur under natural oceanic conditions. Because of the potential toxic effects of too much magnesium, magnesium concentrations in artificial seawater are generally at or below concentrations found in naturally occurring sea water.

Localized delivery of substances to the hair follicle through the use of liposomes has been developed. Liposomes are microscopic globules of lipids which can be manufactured to enclose substances such as medications. The lipid nature of liposomes makes them nonpolar. Sebaceous glands are connected to the hair follicle by ducts and release sebum into the upper third portion of the follicular canal, creating an environment rich in neutral nonpolar lipids. Topically applied liposomes are capable of delivering a wide range of drugs including macromolecules into hair follicles. Experimental evidence of liposomal delivery into the pilosebaceous unit includes quantitative fluorescence with carboxyfluorescein, which is a negatively charged polar compound. Polar compounds such as sea water have been demonstrated to be delivered to the nonpolar follicular environment of the pilosebaceous unit with the use of liposomes.

### BRIEF SUMMARY OF THE INVENTION

It has been identified that artificial sea water, or either artificial or natural sea water to which magnesium has been added to a level of at least 2000 parts per million, can be used to treat acne. The results with added magnesium are far superior to natural sea water in treating acne. Surprisingly, artificial sea water is at least as effective as natural sea water even though it has far fewer constituents than natural sea water. Accordingly, both a product and method of treating the skin with artificial seawater and any sea water with a high concentration of magnesium is the main composition and method of treatment of the present invention.

In one embodiment the present invention comprises a composition for the treatment of acne comprising an artificial sea water, while in yet another embodiment, the invention relates to artificial or natural seawater to which the magnesium concentration is at least 2000 parts per million.

In yet another embodiment of the invention, there is a method of treating the skin comprising applying sea water, which has a concentration of magnesium of at least 2000 parts per million.

In yet another embodiment of the invention, there is a method of treating the skin comprising applying artificial sea water to treat the skin.

In yet another embodiment, there is a composition for the treatment of a skin condition comprising a liposome containing seawater or seawater having a magnesium concentration of at least 2000 parts per million.

In particular, the invention provides:
● A composition suitable for the treatment of the skin comprising an artificial or natural sea water together with additional magnesium, such that the total magnesium concentration in the composition is at least 2000 parts per million.
● A composition comprising (i) artificial or natural sea water together with additional magnesium, such that the total magnesium concentration in the composition is at least 2000 parts per million, or (ii) artificial sea water, for use in medicine.
● A composition comprising (i) artificial or natural sea water together with additional magnesium, such that the total magnesium concentration in the composition is at least 2000 parts per million, or (ii) artificial sea water, for use in (a) the prevention or treatment of a skin condition or (b) wound irrigation or (c) the prevention or treatment of hair loss.
● The use of a composition comprising (i) artificial or natural sea water together with additional magnesium, such that the total magnesium concentration in the composition is at least 2000 parts per million, or (ii) artificial sea water, in the manufacture of a medicament for (a) the prevention or treatment of a skin condition or (b) wound irrigation or (c) the prevention or treatment of hair loss.
● A method of cosmetically treating the skin comprising applying a composition comprising (i) artificial or natural sea water together with additional magnesium, such that the total magnesium concentration in the composition is at least 2000 parts per million, or (ii) artificial sea water, to the skin.
● The use of a composition comprising (i) artificial or natural sea water together with additional magnesium, such that the total magnesium concentration in the composition is at least 2000 parts per million, or (ii) artificial sea water, in the cosmetic prevention or treatment of a skin or hair condition.
● A composition suitable for the treatment of a skin condition comprising a liposome containing i) artificial or natural sea water together with additional magnesium, such that the total magnesium concentration in the composition is at least 2000 parts per million, or (ii) artificial sea water.
● The use of a composition comprising (i) artificial or natural sea water together with additional magnesium, such that the total magnesium concentration in the composition is at least 2000 parts per million, or (ii) artificial sea water, as a sunscreen.
● The use of a composition comprising (i) artificial or natural sea water together with additional magnesium, such that the total magnesium concentration in the composition is at least 2000 parts per million, or (ii) artificial sea water, as a substitute for saline in the treatment of a mammal.

### BRIEF DESCRIPTION OF THE DRAWINGS

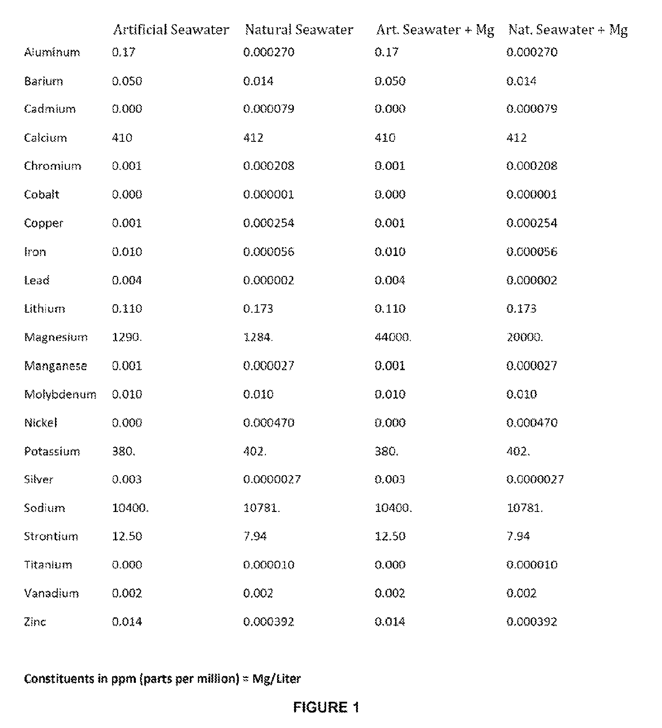

Fig. 1 is a chart showing formulations of natural seawater, artificial sea water, artificial seawater with additional magnesium, and natural seawater with additional magnesium.

### DETAILED DESCRIPTION OF THE INVENTION

The present invention relates to the surprising identification that natural and artificial sea water both show a marked improvement in treating the skin and especially the treatment of acne when there is at least about double the amount of magnesium present when compared to natural sea water and to artificial sea water mimicking natural sea water. It also has been identified that artificial sea water, while not as good as any sea water with increased magnesium, also can be used to treat acne. This is true even though artificial sea water does not contain all the ingredients of natural sea water and has not previously been used to treat acne.

While this invention is susceptible of embodiment in many different forms, there will herein be described in detail specific embodiments, with the understanding that the present disclosure of such embodiments is to be considered as an example of the principles and not intended to limit the invention to the specific embodiments described. This detailed description defines the meaning of the terms used herein and specifically describes embodiments in order for those skilled in the art to practice the invention.

The terms "a" or "an", as used herein, are defined as one as or more than one. The term "plurality", as used herein, is defined as two or as more than two. The term "another", as used herein, is defined as at least a second or more. The terms "including" and/or "having", as used herein, are defined as comprising (i.e., open language)..

Reference throughout this document to "one embodiment", "certain embodiments", and "an embodiment" or similar terms means that a particular feature, structure, or characteristic described in connection with the embodiment is included in at least one embodiment of the present invention. Thus, the appearances of such phrases or in various places throughout this specification are not necessarily all referring to the same embodiment. Furthermore, the particular features, structures, or characteristics may be combined in any suitable manner in one or more embodiments without limitation.

The term "or" as used herein is to be interpreted as an inclusive or meaning any one or any combination. Therefore, "A, B or C" means any of the following: "A; B; C; A and B; A and C; B and C; A, B and C". An exception to this definition will occur only when a combination of elements, functions, steps or acts are in some way inherently mutually exclusive.

As used herein the phrase "artificial sea water" refers to an aqueous solution to which contains a spectrum of minerals, trace elements, organic micronutrients and the like in attempt to, as closely as possible, mimic the composition of naturally occurring sea water. As described above, this means that the magnesium concentration of the sea water, artificial or natural, is around 1300 parts per million or less, such as around 1000 parts per million or less (about 1284 in the chart in Fig. 1). While artificial sea waters may vary in their exact compositions, they are designed in general to be able to support aquatic marine life that normally lives in an ocean setting. These are readily available from aquatic supply houses and the like and are designed for aquariums and the like, or can be prepared from scratch by means well known in the art. ASTM D1141-75 provides a Standard Specification for Synthetic Ocean Water.

It may be, for example, that an artificial sea water is an aqueous solution that includes the following cations in the indicated ppm amounts:
Sodium ions: 8000 to 13000, such as 9000 to 12000, e.g. about 10400
Magnesium ions: 900 to 1500, such as 1000 to 1400, e.g. about 1290
Potassium ions: 300 to 500, such as 350 to 450, e.g. about 380
Calcium ions: 300 to 500, such as 350 to 450, e.g. about 410
Strontium ions: 5 to 20, such as 10 to 15, e.g. about 12.5
Aluminium ions: 0.05 to 0.3, such as 0.1 to 0.25, e.g. about 0.17
Lithium ions: 0.05 to 0.3, such as 0.1 to 0.2, e.g. about 0.11
Barium ions: 0.01 to 0.1, such as 0.02 to 0.07, e.g. about 0.05
Chromium ions: 0 to 0.005, such as 0.0005 to 0.003, e.g. about 0.001
Copper ions: 0 to 0.01, such as 0.0005 to 0.003, e.g. about 0.001
Iron ions: 0 to 0.1, such as 0.005 to 0.03, e.g. about 0.01
Lead ions: 0 to 0.01, such as 0.001 to 0.005, e.g. about 0.004
Manganese ions: 0 to 0.01, such as 0.0005 to 0.003, e.g. about 0.001
Molybdenum ions: 0 to 0.1, such as 0.005 to 0.03, e.g. about 0.01
Silver ions: 0 to 0.01, such as 0.001 to 0.005, e.g. about 0.003
Vanadium ions: 0 to 0.01, such as 0.001 to 0.005, e.g. about 0.002
Zinc ions: 0 to 0.1, such as 0.005 to 0.03, e.g. about 0.014.
In one embodiment, these cations are the only cations included; alternatively in another embodiment if other cations are present they are at a level of 0.0005ppm or less, such as 0.00005ppm or less.
Suitable anions, in particular selected from Cl⁻, SO₄²⁻, HCO₃⁻, Br-, H₂BO₃⁻, F⁻, are also present in artificial sea water. For example, there may be the following cations in the indicated ppm amounts:
Cl⁻ ions 15 000 to 30 000, such as 16 000 to 20 000;
SO₄²⁻ ions 1500 to 5000, such as 2000 to 3000;
HCO₃⁻ ions 100 to 300, such as 100 to 200;
Br- ions 0 to 200, such as 50 to 150;
H₂BO₃⁻ ions 0 to 50, such as 5 to 30;
F⁻ ions 0 to 10, such as 0 to 5.
In one embodiment, these anions are the only anions included; alternatively in another embodiment if other anions are present they are at a level of 0.0005ppm or less, such as 0.00005ppm or less.

It may be that the artificial sea water used includes the constituents set out for the Artificial Seawater in Figure 1, wherein each stated constituent is present in an amount as stated in Figure 1 plus or minus 20%. It may be that the artificial sea water includes the constituents set out for the Artificial Seawater in Figure 1, wherein each stated constituent is present in an amount as stated in Figure 1 plus or minus 10%. It may be that the artificial sea water includes the constituents set out for the Artificial Seawater in Figure 1, wherein each stated constituent is present in an amount as stated in Figure 1 plus or minus 5%.

Natural sea water is of course sea water sourced from a sea or ocean. It may be that the natural sea water used includes the constituents set out for the Natural Seawater in Figure 1, wherein each stated constituent is present in an amount as stated in Figure 1 plus or minus 20%. It may be that the natural sea water includes the constituents set out for the Natural Seawater in Figure 1, wherein each stated constituent is present in an amount as stated in Figure 1 plus or minus 10%. It may be that the natural sea water includes the constituents set out for the Natural Seawater in Figure 1, wherein each stated constituent is present in an amount as stated in Figure 1 plus or minus 5%.

In one embodiment, the sea water contains inorganic salts in a combined concentration of about 35 g/ Kg.

As used herein "magnesium" refers to the magnesium ion and in most cases the Mg⁺⁺ ion that is introduced into the artificial or natural seawater in the form of a magnesium salt. Examples of magnesium salts useful in the present invention are magnesium oxide, magnesium carbonate, magnesium chloride, magnesium sulfate (such as magnesium sulfate heptahydrate, and anhydrous magnesium sulfate) magnesite and dolomite. While the magnesium level in sea water is normally no more than about 1300 parts per million, magnesium is added to the solution in the present invention in an amount to raise the magnesium level in the sample of sea water (natural sea water or artificial sea water) to at least about 2000 parts per million. In some embodiments the magnesium could be at least about 4000 parts per million, 8,000 parts per million, 20,000ppm, 40,000ppm or more. In the embodiment in the example and in Fig. 1, magnesium is present at a level of 44,000 ppm.

In other words, the present invention includes embodiments wherein the concentration of magnesium is at least double the normal naturally occurring magnesium in sea water.

Magnesium is added to the sea water in such a manner that it dissolves the appropriate amount of magnesium at 2000 parts per million and above. Depending on the magnesium salt selected, this could be done at room temperature with stirring or could be done with heat or other means to achieve the desired concentration of magnesium. The optimum amount of magnesium will depend on the particular skin condition being treated, the salt selected, the sensitivity of the patient, the frequency of treatment and the like, however, one skilled in the art with this disclosure could easily optimize the amount of magnesium in view of the present disclosure.

Applying the composition of the present invention can be in any convenient topical method. So, for example, the solution can be applied to the face once a day twice a day or as often as desired to achieve the desired effect. It can be applied and left on or applied and rinsed off or can be used as part of a facial wash in combination with soaps or other skin treatments. Application can be with the hands or by means of the solution applied to cotton balls, cotton swabs, rinsing, or vaporization. In addition, the solution of the present invention could be warmed or otherwise heated so that the pores of the skin will open up during treatment with the present invention.

As used herein "treating the skin" refers to treatment of skin conditions which show improvement with application of the product of the present invention. The present invention is primarily a treatment for acne, but treatment as a preventative for skin conditions is also within the scope of the invention. Conditions of excess oil, other skin infections, eczema, rosacea, psoriasis, seborhhea and the like can also be treated on the skin with the present invention.

The composition of the present invention can also be formulated into a liposome type formulation. As used in the present invention, the term "liposome" means a vesicle composed of amphiphilic lipids arranged in a spherical bilayer or bilayers.

Liposomes are unilamellar or multilamellar vesicles which have a membrane formed from a lipophilic material and an aqueous interior. The aqueous portion contains the present composition to be delivered. Cationic liposomes possess the advantage of being able to fuse to the cell wall. Non-cationic liposomes, although not able to fuse as efficiently with the cell wall, are taken up by macrophages in vivo. Selection of the appropriate liposome depending on the agent to be encapsulated would be evident given what is known in the art.

Liposomes are useful for the transfer and delivery of active ingredients to the site of action. Because the liposomal membrane is structurally similar to biological membranes, when liposomes are applied to a tissue, the liposomes start to merge with the cellular membranes. As the merging of the liposome and cell progresses, the liposomal contents are emptied into the cell where the active agent may act.

Another embodiment also contemplates the use of liposomes for topical administration. Such advantages include reduced side-effects related to high systemic absorption of the administered drug, increased accumulation of the administered drug at the desired target, and the ability to administer a wide variety of drugs, both hydrophilic and hydrophobic, into the skin. Several reports have detailed the ability of liposomes to deliver agents including high-molecular weight DNA into the skin. Compounds including analgesics, antibodies, hormones and high-molecular weight DNAs have been administered to the skin. The majority of applications resulted in the targeting of the upper epidermis.

Liposomes fall into two broad classes. Cationic liposomes are positively charged liposomes which interact with the negatively charged DNA molecules to form a stable complex. The positively charged DNA/liposome complex binds to the negatively charged cell surface and is internalized in an endosome. Due to the acidic pH within the endosome, the liposomes are ruptured, releasing their contents into the cell cytoplasm.

Liposomes which are pH-sensitive or negatively-charged, entrap DNA rather than complex with it. Since both the DNA and the lipid are similarly charged, repulsion rather than complex formation occurs. Nevertheless, some DNA is entrapped within the aqueous interior of these liposomes. PH-sensitive liposomes have been used to deliver DNA, encoding the thymidine kinase gene to cell monolayers in culture. Expression of the exogenous gene was detected in the target cells (Zhou et al., J. Controlled Release, 1992, 19: 269 74).

Another contemplated liposomal composition includes phospholipids other than naturally-derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

"Sterically stabilized" liposomes, which refer to liposomes comprising one or more specialized lipids that, when incorporated into liposomes, result in enhanced circulation lifetimes relative to liposomes lacking such specialized lipids are also contemplated. Examples of sterically stabilized liposomes are those in which part of the vesicle-forming lipid portion of the liposome (A) comprises one or more glycolipids, such as monosialoganglioside GM1, or (B) is derivatized with one or more hydrophilic polymers, such as a polyethylene glycol (PEG) moiety. While not wishing to be bound by any particular theory, it is thought in the art that, at least for sterically stabilized liposomes containing gangliosides, sphingomyelin, or PEG-derivatized lipids, the enhanced circulation half-life of these sterically stabilized liposomes derives from a reduced uptake into cells of the reticuloendothelial system (RES) (Allen et al., FEBS Lett., 1987, 223: 42; Wu et al., Can. Res., 1993, 53: 3765).

Many liposomes comprising lipids derivatized with one or more hydrophilic polymers, and methods of preparation thereof, are known in the art. See, e.g., Sunamoto et al. (Bull. Chem. Soc. Jpn., 1980, 53: 2778) described liposomes comprising a nonionic detergent, 2C12 15G that contains a PEG moiety. Illium et al. (FEBS Lett., 1984, 167: 79) noted that hydrophilic coating of polystyrene particles with polymeric glycols results in significantly enhanced blood half-lives. Synthetic phospholipids modified by the attachment of carboxylic groups of polyalkylene glycols (e.g., PEG) are described by Sears (U.S. Pat. Nos. 4,426,330 and 4,534,899). Klibanov et al. (FEBS Lett., 1990, 268: 235) described experiments demonstrating that liposomes comprising phosphatidylethanolamine (PE) derivatized with PEG or PEG stearate have significant increases in blood circulation half-lives. Blume et al. (Biochimica et Biophysica Acta, 1990, 1029: 91) extended such observations to other PEG-derivatized phospholipids, e.g., DSPE-PEG, formed from the combination of distearoylphosphatidylethanolamine (DSPE) and PEG. Liposomes, having covalently bound PEG moieties on their external surface, are described in European Patent No. EP 0 445 131 BI and WO 90/04384 to Fisher. Liposome compositions containing 1 20 mole percent of PE derivatized with PEG, and methods of use thereof, are described by, e.g., Woodle et al. (U.S. Pat. Nos. 5,013,556 and 5,356,633) and Martin et al. (U.S. Pat. No. 5,213,804 and European Patent No. EP 0 496 813 B1). Liposomes comprising a number of other lipid-polymer conjugates are disclosed in WO 91/05545 and U.S. Pat. No. 5,225,212 (both to Martin et al.) and in WO 94/20073 (Zalipsky et al.). Liposomes comprising PEG-modified ceramide lipids are described in WO 96/10391 (Choi et al.). U.S. Pat. No. 5,540,935 (Miyazaki et al.) and U.S. Pat. No. 5,556,948 (Tagawa et al.) describe PEG-containing liposomes that can be further derivatized with functional moieties on their surfaces.

### Examples

Four aqueous formulations, as described in Fig. 1, are either made, purchased (in the case of artificial sea water) or collected (in the case of natural sea water). To make the latter two compositions with additional magnesium, magnesium is added to the sea water in the form of the magnesium chloride salt sufficient to reach the indicated concentrations of magnesium ion. Individuals are treated with one of the four formulations for conditions of acne and the result after daily treatments over four weeks are compared. Compositions comprising natural and artificial sea water are comparable, with artificial sea water performing slightly better than natural. Individuals treated with either artificial or natural sea water with additional magnesium show marked improvement compared with the compositions without addition of magnesium.

The present invention compositions have also been determined to be useful for the treatment of skin wrinkles. Skin wrinkles are one aspect of skin aging. Skin aging is known to be from both intrinsic factors and photoaging factors. Intrinsic factors include all clinical, histologic and physiologic changes in the sun-protected skin of adults. These include a decline in the ability to repair damaged DNA and abnormalities in transepidermal water loss. It is the water loss and effects of dehydration that contribute to the appearance of skin wrinkles. Photoaging has a major impact on appearance causing wrinkles, hyperpigmentaion and a leathery look. There are exaggerated functional losses as seen in intrinsic aging with further loss of immune function and inflammation as a result of ultraviolet radiation.

Synthetic sea water appears to be useful in the treatment of aging skin through many mechanisms. While not wanting to be held to a particular theory, the following seems to explain the results obtained with treatment with synthetic sea water. These include anti-inflammatory support, anti-oxidant activity, enhanced molecular and cellular detoxification and scavenging, immunostimulation, as well as osmotic effects of cellular water.

The beneficial anti-inflammatory and antioxidant effects of synthetic sea water appear to be mediated though the apparent supplementation of trace minerals. Trace mineral deficiency is common and is from a combination of poor nutrition and depleted soils. Trace mineral deficiency is well known to have adverse effects on the human anti-inflammatory and anti-oxidant biochemical pathways. These have been extensively studied with zinc and selenium as common examples. The importance of selenium as an essential trace element has been published in biomolecular chemistry journals reporting the mechanism through which selenium exerts its redox activities. There are several selenocysteine-containing enzymes such as glutathione peroxidase, iodothyronine deiodinase and thioredoxin reductase. All these enzymes have selenocysteine in the active sites.

Selenium's role as an essential nutrient is as a result of its unique chemistry enabled by the presence of selenium in selenoproteins. Epidemiological findings have linked inadequate status of selenium to increased risk of cancer. The protective action of selenium is a combination of various mechanisms. Amongst all the diverse mechanisms that have been proposed some important ones are (a) the protective role of selenoproteins/selenoenzymes, (b) induction of apoptosis, (c) immune system effects, (d) detoxification of antagonistic metals, (e) inactivation of nuclear transcription factor, (f) regulation of lipoxygenases, (g) effect on advanced cancer condition, (h) reduction of oxidative stress, (i) induction of Phase II enzymes, (j) androgen receptor down regulation, (k) inhibition of DNA adduct formation, and (I) cell cycle arrest. Many of these effects are directly applicable to the benefits concerning the cellular molecular biology of skin.

Other enzymes with anti-inflammatory and antioxidant effects which use zinc and selenium as co-factors include superoxide dismutase, glutathione peroxidase and malondialdehyde. This is not a comprehensive list of enzymes involved in the anti-inflammatory and antioxidant biochemical pathways but are used as examples. Many more enzymes are involved and each has trace minerals as cofactors. Selenium and zinc are not the only two cofactors and synthetic sea water has many trace elements and minerals. The important concept is that inflammation and oxidative damage are components of a complex process with skin aging which results in skin wrinkles. Synthetic sea water has been determined to replenish many trace minerals and elements which are essential cofactors for enzymatic function which helps explain the novel benefits observed with artificial seawater treatments of skin.

Nitric oxide is another enzymatically produced substance with beneficial effects on skin aging. It is another antioxidant, but has many other properties as well. The effect of nitric oxide on epithelial cells has been studied. In burn wounds there is enhanced re-epithelialization though follicle stem cell recruitment, increased number of procollogen-expressing fibroblasts, increased angiogenesis, enhanced hair follicle regeneration, and promotion of wound bed infiltration and retention with growth factors and cytokines during the healing process. Nitric oxide is produced enzymatically by nitric oxide synthetase and requires co-factors as with other enzymes. There is experimental evidence in human skin fibroblasts that nitric oxide protects against the injurious effects of ultraviolet A radiation. This nitric oxide dependent mechanism was confirmed by the observation in nitric oxide depleted cultures of increased susceptibility to ultraviolet A induced lipid peroxidation. The molecular mechanisms of endothelial dysfunction from nitric oxide synthetase inhibition have also been published in the medical literature. These effects of nitric oxide are a result of nitric oxide production though enzymatic function of nitric oxide synthetase and are dependent upon trace elements for cofactors and appear to be supported by topical administration of synthetic sea water.

Molecular detoxification is also involved in skin aging. There is an age dependent decline in the activity of the hydrogen peroxide detoxifying antioxidant catalase in chronically sun-exposed epithelium. Catalase mimetics, as well as peroxynitrite scavengers, are thought to maintain hydrogen peroxide detoxification pathways. Creatinine kinase activity is also lost through oxidative stress. These examples of additional enzymes involved in the detoxification process support the concept of micronutrient and trace mineral supplementation for optimal enzymatic activity to prevent loss of skin homeostasis and could be another explaination of the mechanism that artificial seawater treats skin wrinkles.

Another mechanism of protection against epidermal ultraviolet B photodamage, as well as water loss, is mediated though caspase-14. Caspase-14 is an aspartate-specific proteinase whose expression is restricted almost exclusively to the suprabasal layers of the epidermis and the hair follicles. The proteolytic activation of caspase-14 is associated with stratum corneum formation indicating caspase-14 is essential for keratinocyte differentiation and cornification. Caspase-14 is dependent upon a proteinase enzyme for formation and has beneficial effects in protection against ultraviolet B radiation by controlling the ultraviolet B scavenging capacity of the stratum corneum.

Caspase-14 has an additional mechanism which is beneficial in the treatment of wrinkles by increasing cell water. This effect is seen experimentally as caspase-14 deficient epidermis is characterized by reduced skin-hydration levels and increased water loss. Cellular biology has revealed the mechanism as being mediated by the ability of caspase-14 to cleave profilaggrin. This mechanism is supported by the observation that altered profilaggrin processing patterns have been linked to caspase-14 deficient epidermis. The importance of the filaggrin structure in moisturization of the skin has been established and is another example of an enzymatically dependent pathway involved in the prevention of skin aging. Once again artificial sea water may be supplying the necessary elements to achieve reduction of wrinkles by this method.

The severity of skin wrinkles is usually considered related to cellular water. Skin is the limiting tissue of the body and within skin, the stratum corneum (SC) of the epidermis is the limiting barrier to water loss. Water homeostasis of the epidermis is important for the appearance and physical properties of the skin, as well as for water balance in the body. The transport of water is dependent upon gap junctions and cellular membrane channels. Aquaporin-3 (AQP3) is a membrane transporter of water expressed in plasma membranes in the basal layer keratinocytes of epidermis in normal skin. Gap junctions and plasma membrane channels are composed of proteins. The tertiary structure of proteins is dependent upon the chemical environment and the permeability is sensitive to electrolyte ion concentrations. For example, the permeability of gap junctions is regulated by calcium ion. Likewise, calcium membrane pumps are regulated by magnesium ion concentrations. Intracellular water transport and homeostasis is maintained by membrane transporters and channels and these are made of proteins whose structure and function are dependent upon the chemical environment composed of elements and minerals.

There is also an osmotic component to skin wrinkles where severity of wrinkles is decreased with increased cellular water and the amount of cellular water can be increased along osmotic concentration gradients. Topical application of synthetic sea water would be expected to increase cellular water through this mechanism in addition to the biochemical and cellular molecular mechanisms previously discussed. Transport across liquid membranes is described by Fick's Law, the Nerst-Planck equation or the Einstein equation to predict diffusivity depending upon electrochemical potentials and concentrations. Essentially, the permeability is a function of the solute concentration, the area of contact and the contact time.

These mechanisms of anti-inflammatory support, anti-oxidant activity, enhanced molecular and cellular detoxification and scavenging, immunostimulation, as well as osmotic effects of cellular water are all supported with the topical administration of synthetic sea water. This is the cellular molecular basis of the benefits of synthetic sea water with added magnesium for applications associated with skin homeostasis. One such application would be for the treatment of skin wrinkles.

It has also been discovered that the present invention relates to the use of the present invention for use as a sunscreen. The mechanism for this use is postulated to be similar as for skin wrinkles with special emphasis on the antioxidant properties. Antioxidants are known to decrease the severity of ultraviolet damage and to promote healing of epithelial tissues.

The present invention, artificial sea water, is also useful for the treatment of hair loss. Hair follicle stem cell recruitment and enhanced hair follicle regeneration is seen with nitric oxide as has been discussed. In addition, Caspase-14 is an aspartate-specific proteinase whose expression is restricted almost exclusively to the suprabasal layers of the epidermis and the hair follicles. Each of the pilosebaceous units discussed concerning acne vulgaris also contains a hair follicle and improvements in pilosebaceous unit function would also be expected to improve hair growth.

In addition to these cosmetic uses there are many medical uses of synthetic sea water with added magnesium. Normal saline is commonly used for medical applications. Synthetic sea water would be superior in these applications as the benefits of the sodium and chloride are maintained and there are additional benefits from the trace minerals and elements. The benefits are similar to those described concerning the cellular molecular biology of skin.

A major medical application of the present invention is application to a wound for wound irrigation. Wound irrigation is a common medical procedure and the choice of cleansing solutions has been extensively evaluated. It has been determined that a wound cleansing solution should meet a number of important characteristics such as described in Flanagan, M. (1997Wound cleansing (Chapter 5). In: Morison, M., Moffat, C., Bridel-Nixon, J., Bale, S. (eds). Nursing Management of Chronic Wounds. Mosby, Lond*on) and incorporated herein by reference.*
- Is non-toxic to human tissues,
- Remains effective in the presence of organic material,
- Is able to reduce the number of micro-organisms,
- Does not cause sensitivity reactions,
- Is widely available,
- Is cost-effective, and
- Is stable; with a long shelf life.

Normal saline fulfils all the criteria given above and has been identified as the only completely safe cleansing agent and the treatment of choice for use on most wounds.

Wound care continues to be a major medical problem and has become a topic of special interest with The Journal of Wound Care as a forum for discussion. Improvement in wound irrigation fluids is felt to be desirable and there has been discussion that a more complex isotonic solution may provide a further improvement. The present invention, therefore, has applicability to wound management as a new solution for wound irrigation.

In addition to wound irrigation, the present invention would be useful for peritoneal lavage, sinus surgery, cystoscopy, ureteroscopy, colonoscopy, bronchoscopy, laryngoscopy, hysteroscopy, arthroscopy, an eye lubricant or irrigant, a source of water and electrolytes, priming for dialysis, and IV fluids. Normal saline is used for many of these indications because of improved visualization. Synthetic sea water would have an added advantage by promoting healing of the tissues being treated and the mechanism of improved tissue healing is multifactorial as discussed.

These same mechanisms of anti-inflammatory support, anti-oxidant activity, enhanced molecular and cellular detoxification and scavenging, immunostimulation, as well as osmotic effects of cellular water, which are all supported with the topical administration of synthetic sea water with added magnesium, would support the use of synthetic sea water with added magnesium in medical applications where normal saline is currently used.

The concentration could be adjusted for individual uses. For example, a concentrated solution would be more beneficial for use for skin wrinkles. A diluted solution would have applicability for eye irrigation. A general formula, nX could be used where X is the standard concentration as listed in Fig. 1 and n is a fraction when diluted solution is need for sensitive tissues such as the eye or for intravenous use. A value of n greater than one would be concentrated solutions such as for use with skin wrinkles.

## Claims

1. A composition suitable for the treatment of the skin comprising an artificial or natural sea water together with additional magnesium, such that the total magnesium concentration in the composition is at least 2000 parts per million.

2. A composition comprising (i) artificial or natural sea water together with additional magnesium, such that the total magnesium concentration in the composition is at least 2000 parts per million, or (ii) artificial sea water, for use in medicine.

3. A composition comprising (i) artificial or natural sea water together with additional magnesium, such that the total magnesium concentration in the composition is at least 2000 parts per million, or (ii) artificial sea water, for use in (a) the prevention or treatment of a skin condition or (b) wound irrigation or (c) the prevention or treatment of hair loss.

4. The use of a composition comprising (i) artificial or natural sea water together with additional magnesium, such that the total magnesium concentration in the composition is at least 2000 parts per million, or (ii) artificial sea water, in the manufacture of a medicament for (a) the prevention or treatment of a skin condition or (b) wound irrigation or (c) the prevention or treatment of hair loss.

5. The composition of claim 3 or use of claim 4, wherein the skin condition is selected from the group comprising acne, eczema, rosacea, seborhhea and psoriasis.

6. A method of cosmetically treating the skin comprising applying a composition comprising (i) artificial or natural sea water together with additional magnesium, such that the total magnesium concentration in the composition is at least 2000 parts per million, or (ii) artificial sea water, to the skin.

7. The use of a composition comprising (i) artificial or natural sea water together with additional magnesium, such that the total magnesium concentration in the composition is at least 2000 parts per million, or (ii) artificial sea water, in the cosmetic prevention or treatment of a skin or hair condition.

8. The use of claim 7 wherein the condition is skin wrinkles, skin having excess oil, or hair loss.

9. The invention according to any one of claims 1 to 8, wherein the magnesium concentration in the composition is at least 8000 parts per million.

10. The invention according to claim 9, wherein the magnesium concentration in the composition is at least 40,000 parts per million.

11. The invention according to any one of claims 1 to 10, wherein the magnesium is present in the form of at least one of magnesium oxide, magnesium carbonate, magnesium chloride, magnesium sulfate, magnesite and dolomite.

12. A composition suitable for the treatment of a skin condition comprising a liposome containing i) artificial or natural sea water together with additional magnesium, such that the total magnesium concentration in the composition is at least 2000 parts per million, or (ii) artificial sea water.

13. The use of a composition comprising (i) artificial or natural sea water together with additional magnesium, such that the total magnesium concentration in the composition is at least 2000 parts per million, or (ii) artificial sea water, as a sunscreen.

14. The use of a composition comprising (i) artificial or natural sea water together with additional magnesium, such that the total magnesium concentration in the composition is at least 2000 parts per million, or (ii) artificial sea water, as a substitute for saline.

15. The use of claim 14 wherein the composition is used as a substitute for saline in peritoneal lavage, sinus surgery, cystoscopy, ureteroscopy, colonoscopy, bronchoscopy, laryngoscopy, hysteroscopy, arthroscopy, an eye lubricant or irrigant, a source of water and electrolytes, priming for dialysis, or IV fluids.
